# EUROPEAN PATENT APPLICATION

(11) **EP 3 412 321 A1**
(43) Date of publication of application: **12.12.2018**
(21) Application number: 16873479.6
(22) Date of filing: 25.01.2016
(51) Int. Cl.: A61L 27/02

(54) **IMPLANT**

(30) Priority: 07.12.2015 UA 201512141
(71) Applicant: Mishchenko, Oleg Nikolaevich, Zaporozhskaya obl. 69089 (UA)
(72) Inventor: Mishchenko, Oleg Nikolaevich, Zaporozhskaya obl. 69089 (UA)
(74) Representative: Karczmitowicz, Teresa Ewa
(86) International application number: PCT/UA2016/000011
(87) International publication number: WO 2017/099696

(57) **Abstract**

The invention relates to the field of medical technology and may be used in preparing implants for dentistry, traumatology, orthopedics, vascular surgery and various forms of plastic surgery. Essence of the invention: an implant containing a base which is covered in an oxide film, the surface of which contains grooves in an area which comes into contact with soft tissue, said grooves being applied via laser. The invention is novel in that the groove dimensions are 5-300nm, and in that the grooves are disposed in a given direction in accordance with the anatomical location and functional application of the implant, at an interval of 5-400nm. Technical result: improving adhesion and the subsequent integration of the cellular elements of soft tissue into the structure of the oxide film, thus achieving a stable, strong connection of soft tissue to the surface of an implant, the connection not changing under the effects of a functional load, which in turn allows for broadening the functional capabilities of the claimed implant.

## Description

Implant which is a subject of the present invention belongs to the discipline of medical technique and it may be used for manufacturing of implants in dentistry, traumatology, orthopaedics, vascular surgery and various types of plastic surgery, in particular where a balanced, permanent and quick integration of the implant to the soft tissue is required. The implant made according to the present invention enables a significant improvement of adhesion and subsequently cell integration of the soft tissues elements into the structure of oxide layer; it also enables to achieve a balanced and permanent incorporation of soft tissues into the implant surface, which significantly increases functionality of the implant in comparison to the current state of the art.

It is known from registration of the Russian invention (see patent no RU2485979 of 25.08.2009, patented on 27.06.2013, A61L27/04, A61L27/06, A61L27/30, A61L31/08, A61L31/16, A61F2/28) and extension thereof in the European procedure number EP2371398 - the method of manufacturing medical agents, including implants containing an external oxygen coating. The surface of oxygen coating has pores of the 1-10 µm. To enhance antibacterial properties, the pores are impregnated with iodine or its compounds. An iodine compound is favourably a polyvinylpyrrolidone iodide, ²-cyclodextrin iodide or silver iodide. Furthermore, the basic material is *favourably a* metallic material and any of the Ti alloys, Ti, stainless steel and Co-Cr alloy having biological compatibility.

The porous coating on the material surface (implant) may be created by means of any method - electrochemical, chemical, thermal and/or mechanical processing or a combination of two or more of the aforementioned processing methods.

The implant made according to the quoted invention is characteristic of a very good antimicrobial activity and durability of antimicrobial activity and biocompatibility however, a major defect of this solution is poor integration to soft tissues (in zones where the implant leaves the bone), which reduces its functionality.

The implant most similar to the one which is applied for - both in relation to the technical essence and achieved results - is an implant with a base covered with oxygen coating whose surface in the zone of interface with the soft tissue contains laser made grooves (see Nevins Myron at all. International Journal of Periodontics & Restorative Dentistry, 2008; 28:111-121). The groove size is 2-12 µm, optimally 6-12 µm.

In the known patent, the micro-grooves hinder apical migration of the epithelial tissue and fibroblasts, allowing slowly growing osteoblasts to reach the surface and fix to it, which fosters obtaining a strong fixing of the connective tissue. Weaknesses of the discussed implant is insufficient adhesion to soft tissues and therefore, lack of proper integration of soft tissues to the implant surface.

The purpose of the present invention is to manufacture an implant to be used in dentistry, traumatology, orthopaedics, vascular surgery and various types of plastic surgery; the implant which ensures excellent adaptation of its surface to the fibroblast structure in different stages of their growth and maturation and provides a prefect integration of soft tissues to the implant surface.

Below, a simplified summary of the invention is presented. This summary is not an extensive explanation of the invention, and its only purpose is presentation of the invention concept in a simplified form as an introduction to a more detailed description which is presented below.

External surface of the implant made according to the invention is covered by the commonly used oxygen coating. On the said oxygen coating surface, in the zone of interface with the soft tissue, laser made nanopores of 5-300 nm size are placed. They are made in the determined direction depending on anatomic location and intended functionality of the implant, with the distance between nanopores of 5-400 nm. Technical effect of the invention is an improvement of adhesion and then integration of cell elements of soft tissues into the oxygen layer structure, thanks to which the incorporation of soft cells into the implant surface is durable and strong and not changing under the working load, which significantly broadens functionality of the implant.

The subject of the invention is an implant, in which thanks to a novel making of nanopores on the implant surface covered with the currently used oxygen coating, adhesion is improved as well as the integration of cell elements of soft tissues into the oxygen layer structure, thanks to which a durable, strong and not changing under the working load incorporation of soft tissues into the implant surface is achieved. According to the invention, dimensions of nanopores are of 5-300 nm, nanopores are placed in a specific direction, depending on functional purpose of the implant, and interval between them is 5-400 nm.

A series of laboratory research (which shall be further discussed below) confirmed that there is a cause and effect relationship between the properties of the present invention and the test results, proving excellent results achieved in relation to significant improvement of adhesion and integration of cell elements of soft tissues into the oxygen layer structure of the implant whose:
- nanopores are of 5-300 nm dimension;
- nanopores are made in the direction compliant with the selected intended use of the implant (i.e. where the implant is to be placed);
- nanopores are made in the interval of 5-400 nm.

It is known that in wound healing, hence also in integration of soft tissues with the implant surface, an active role is played by fibroblasts - main cells of the connective tissue; fibroblasts are of mesenchymal origin, and morphologically they are round or elongated spindle shaped cells with attachments (pseudopods, pods) and a flat elliptical nucleus.

Fibroblasts synthetize tropocollagen, collagen precursor, intercellular matrix and main substance of the connective tissue, an amorphic substance similar to gel, filling the space between cells and fibres of the connective tissue. Structure and size of fibroblasts is non-identical on different stages of their growth and maturation. Direction in which nanopores are made depends on the structure of fibroblasts and the size of nanopores depends on the size of fibroblasts.

Presence of nanopores on the surface of the implant oxygen coating in the zone of interface with a specific soft tissue (i.e. tissue surrounding the place where the implant is required), placed in a specific direction, depending on anatomic location and implant function in the interval of 5-400 nm, enables to obtain a surface optimally resembling the structure of fibroblasts on different stages of their growth and maturation and it facilitates binding of protein fractions and ensures adaptative reaction of the fibrous (scar) tissue on various stages of its formation. In consequence, the adhesion is improved as well as integration of cell elements of soft tissues into the oxygen layer structure, thanks to which a durable, strong and not changing under the working load incorporation of soft tissues into the implant surface is achieved.

Laser making of nanopores provides the possibility to precisely manage the nanopore size and their location on the implant surface, which depends on fibroblast size and shape, anatomically relevant. It is the possibility of managing the direction of nanopores on the implant surface which enables to ensure regeneration focused on the cell structures growth, depending on anatomic location and intended use of the implant, which significantly increases functional properties of the implant according to the invention.

In view of the aforementioned, if the implant is used in dentistry practice, it is purposeful to place nanopores directed vertically towards the surface of oxygen coating on the cervical margin of a tooth implant (zone of the implant interface with mucosa). At the same time, a durable incorporation of soft tissues with the implant surface is provided, and infestation of microorganisms is reduced due to hindrance of microorganisms growth.

In the case implants are used in vascular surgery (filters for the inferior vena cava, vascular occluders, stents), it is necessary to place nanopores in the form of a mesh (transversal nanopores) on the surface of the implant oxygen coating in the zone of interface with the soft tissues. This ensures durable connection of soft tissues with the implant surface, not changing under the working load, which reduces the effect of tension and micro-friction in the tissue structure.

Dimensions of nanopores according to the invention and intervals between them were determined as optimal on the basis of a series of experiments. The research confirmed that nanopore dimensions and size of interval between them enable to optimally use relevant structure of fibroblasts on different stages of their growth and maturation, so that adhesion is significantly improved thus enabling excellent integration of cell elements of soft tissues into the oxygen coating structure. Therefore, the implant according to the invention enables to obtain a durable, strong and not changing under the working load connection of soft cells with the implant surface.

The implant according to the invention is manufactured by means of the following method.

The following may be used to manufacture the implant base: titanium and its alloys, zirconium and its alloys, titanium-zirconium alloys with different alloy compounds, zirconium oxide, and aluminium oxide. The oxygen coating on material of the base is produced in natural oxidation conditions and by means of pre-treatment in oxygen environment.

After initial polishing, nanopores of 5-300 nm size are produced by means of a laser, on the surface of the oxygen coating, in the zone of interface with the soft tissue. Nanopores are localised in a determined direction, depending on anatomic location and intended use of the implant, with the 5-400 nm interval.

Testing of the soft tissue incorporation into the implant which is a subject of the present invention, was conducted in laboratory conditions and compared to the control (polished) sample and prototype sample.

Test results are presented in the figures, where:
- figure 1 - presents an image of the sample surface after polishing, obtained by means of atomic force macroscopy;
- figure 2 - presents surface of the sample according to the invention, after laser treatment, obtained by means of atomic force macroscopy;
- figure 3 - presents surface of the control sample after 10 days of contact with the soft tissue;
- figure 4 - presents surface of the prototype sample;
- figure 5 - presents surface of the sample applied for, after 10 days of installation, contact with the soft tissue.

The basic material of the tested samples was titanium, zirconium and their alloys (the figures depict the results of tests made on samples whose base was made of zirconium). The oxygen coating on material of the base was made by means of pre-treatment in the oxygen environment.

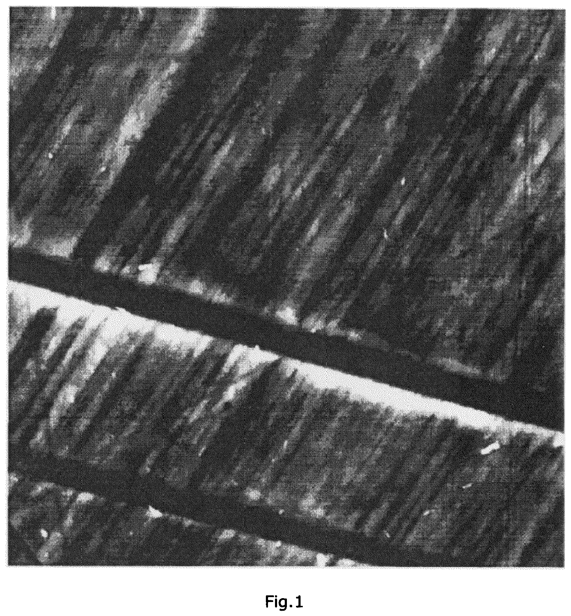

The oxygen coating surface was preliminary polished to roughness 15, as shown in Fig.1.

Groups of samples applied for were treated by means of the Yb femtosecond laser of the 1,030 nm wavelength, E II V polarisation, 213 fs impulse duration, 150-300 mW power (depending on material of the base).

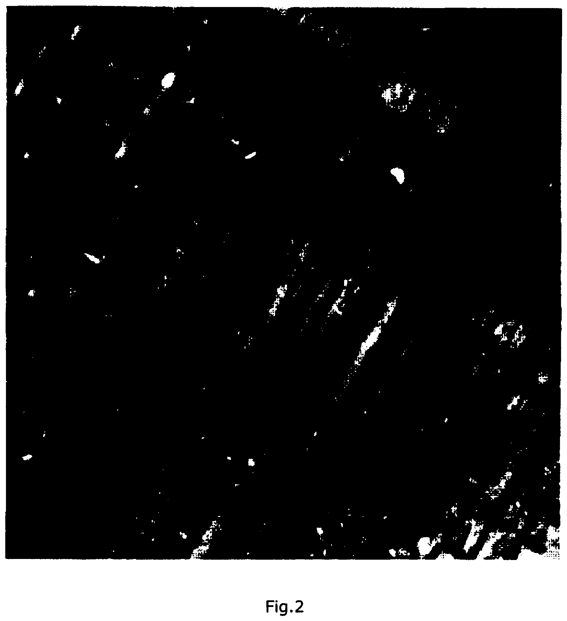

As a result of the Yb-laser treatment, on the oxygen coating surface of the applied for samples nanopores appeared of the 5-300 nm size, localised within the range 5-400 nm (as in Fig. 2).

Experimental testing of the soft tissue cells reaction on the surface of the tested samples was made on mice. For this purpose, samples were placed under the mice skin according to the standard method (in the area of the withers). On day 10, the samples were removed and tested by a scanning microscope.

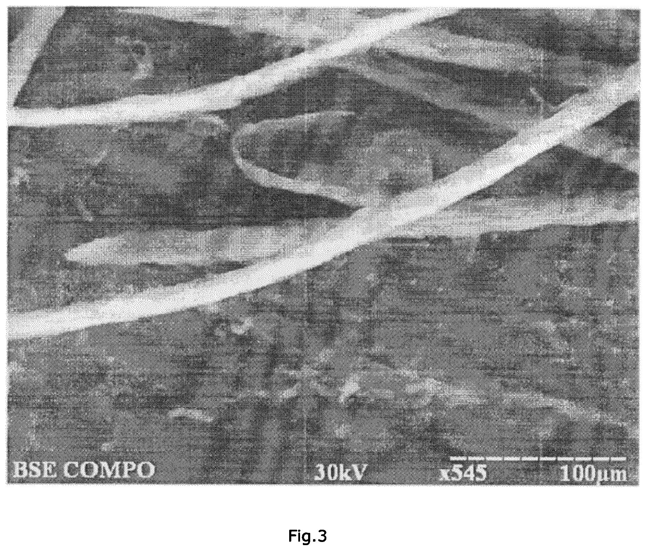

The research showed that single collagen fibres appeared on the surface of the control sample; no correlation was noted between the fibres and the surface of the sample (as in Fig. 3).

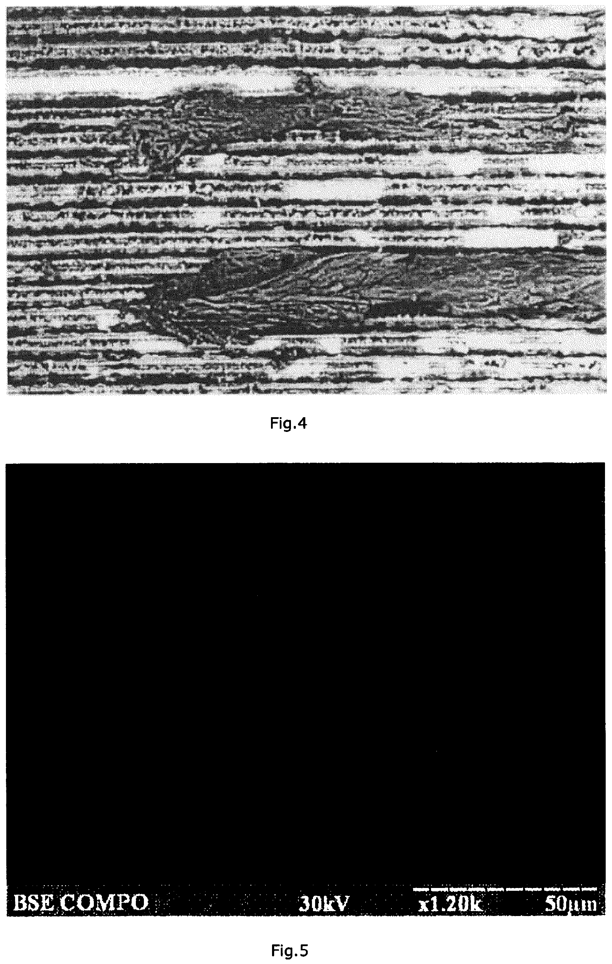

On the surface of the prototype sample single fibroblasts are noticed, traces of protein fractions, split without a distinct fixing on the surface of the sample (as in Fig. 4).

In the group of the applied for samples noticeable pseudopods of fibroblasts appear, which get actively fixed to the surface, i.e. integration of the soft tissue with surface of the sample was visually observed (as in Fig. 5).

In this way, data from the experiments performed confirm increased resistance and durability of the soft tissues integration in samples which were subject of the experiment in comparison to the prototype sample.

Similar results were obtained while testing samples whose base was made of titanium and alloys of titanium and zirconium.

The implant according to the invention significantly improves adhesion and integration of cell elements of soft tissues into the oxygen layer structure, and thanks to that the incorporation of soft tissues into the implant surface is not changed under the impact of working load.

## Claims

1. The implant with a base covered by oxygen coating whose surface in the zone of interface with the soft tissues contains laser made nanopores characteristic in that the size of nanopores is 5-300 nm, and nanopores are localised in the determined direction, depending on anatomic location and intended use of the implant, in the interval of 5-400 nm.
